**Europäisches Patentamt**

(19) **European Patent Office**

**Office européen des brevets**

(11) Publication number : **0 383 922 B1**

(12) **EUROPEAN PATENT SPECIFICATION**

(45) Date of publication of patent specification :
09.11.94 Bulletin 94/45

(51) Int. Cl.[5] : **G01N 33/12,** C12Q 1/26,
C12Q 1/34, C12Q 1/42,
C12Q 1/48

(21) Application number : 89902539.9

(22) Date of filing : 17.02.89

(86) International application number :
PCT/JP89/00160

(87) International publication number :
WO 89/07655 24.08.89 Gazette 89/20

(54) METHOD FOR MEASURING DEGREE OF FRESHNESS AND MEASURING KIT FOR THE METHOD.

(30) Priority : 18.02.88 JP 36047/88
26.12.88 JP 328329/88

(43) Date of publication of application :
29.08.90 Bulletin 90/35

(45) Publication of the grant of the patent :
09.11.94 Bulletin 94/45

(84) Designated Contracting States :
AT BE CH DE FR GB IT LI LU NL SE

(56) References cited :
EP-A- 0 137 677
JP-A- 5 913 200
JP-A- 6 322 196
JP-A-57 163 497
CHEMICAL ABSTRACTS, vol. 105, no. 21, 24
November 1986, Columbus, OH (US); p. 613,
no. 189728u
CHEMICAL ABSTRACTS, vol. 100, no. 5, 30
January 1984, Columbus, OH (US); F. UDA et
al., p. 354, no. 33311p

(73) Proprietor : DAIICHI PURE CHEMICALS CO.
LTD.
13-5, Nihonbashi 3-chome
Chuo-ku Tokyo103 (JP)

(72) Inventor : OKUSA, Naoya, Tokyo Research
Laboratory
Daiichi P. Chem. Co.Ltd.,
5-12, Narihira 5-chome
Sumida-ku, Tokyo 130 (JP)
Inventor : UCHIYAMA, Hitoshi
27-15, Shinoharanishi-machi
Kouhoku-ku
Yokohama-shi Kanagawa-ken 222 (JP)
Inventor : NOZAKI, Yoshitaka
1-42-4, Matsugoaka
Funabashi-shi
Chiba-ken 274 (JP)

(74) Representative : Wächtershäuser, Günter,
Prof. Dr.
Patentanwalt
Tal 29
D-80331 München (DE)

EP 0 383 922 B1

## Description

### Field of the Invention

The present invention relates to a method for judging freshness, and, in particular, to a method for quickly and easily measuring ATP-related compounds, the production of which accompanies any deterioration in the freshness of seafoods and meats.

### Description of the Prior Art

In recent years, in the distribution of seafoods and meats, especially in the distribution industry, there has been a strong demand for the objective determination the freshness of the products.

Conventionally, measuring an amount of ATP decomposition products has been known as a guideline for judging the freshness of seafoods. Specifically, ATP decomposes into ADP, AMP, inosinic acid, inosine, and hypoxanthine, in that order, and these compounds are accumulated in the fish meat. The K value, which is the ratio of the weight of inosine and hypoxanthine to the total weight of ATP-related compounds, is regarded as a most accurate guideline for the judgment of freshness. Methods of measuring the K value which have been developed and used are the ultraviolet absorption method, the visible light absorption method, the oxygen electrode method, and the like. Among these, the visible light absorption method uses a system which decomposes all ATP-related compounds into uric acid see for example NEW FOOD IND. (1983), 25(11), p 2-7. Hydrogen peroxide ($H_2O_2$) is produced along with uric acid in an enzyme reaction system. The amount of $H_2O_2$ is colorimetrically determined in the visible light absorption method. In this method, a color proportional to the amount of ATP-related compounds develops by adding and reacting a catalase and a color reagent, for example, 4-aminoantipyrine (coupler) and phenol, dimethylaniline or diethyl-m-toluidine (developer). The K value can be obtained by measuring the color by a spectrophotometer.

The advantage of this method is that the colorimetry does not require measuring the absorption of ultraviolet light, thus ensuring the use of less expensive measuring instrument. In the UV light method, if ultraviolet absorbing compounds other than ATP-related compounds are present, errors are produced in the judgment of the freshness of the product. The visible light absorption method is a superior method for judging freshness of such a product.

However, in the visible light absorption method outlined above, all the reagents must be prepared at the time they are used, which gives rise to many problems from the aspect of operating efficiency and the like. A method of impregnating a test strip with a color developer and drying it has been considered to simplify this visible light absorption method. However, since color developers used conventionally, such as phenol, dimethylaniline, diethyl-m-toluidine, and the like, are all oil-type materials and are therefore difficult to apply to this method. In addition, the sensitivity of the test strips is poorer than the sensitivity of solutions.

The present inventors have conducted extensive studies in order to develop a method for judging the freshness of seafoods and meats which is free from the inconvenience of preparing the reagents when they are to be used and which can be easily and quickly carried out on site, using a small, convenient device. As a result, the inventors found that the use of N-ethyl-N-sulfobutyl-m-toluidine as a color developer combined with 4-aminoantipyrine as a developer in the visible light absorption method was able to detect ATP-related compounds with more sensitivity than conventional color developers such as phenol, dimethylaniline, and diethyl-m-toluidine. The inventors further found that a test strip could easily be impregnated with N-ethyl-N-sulfobutyl-m-toluidine and dried, and could provide a satisfactory sensitivity in the freshness determination.

### Disclosure of the Invention

The present invention provides a method for measuring freshness which comprises:
(i) a step for neutralizing a test sample containing ATP-related compounds;
(ii) a step for reacting a prescribed amount of the test sample neutralized in step (i) and an enzyme which can decompose ATP-related compounds into hydrogen peroxide and uric acid, and contacting the reaction liquid with a test strip impregnated with N-ethyl-N-sulfobutyl-m-toluidine, thus developing a color in the test strip;
(iii) a step for reacting a prescribed amount of the test sample neutralized in step (i) and an enzyme which can decompose inosine and hypoxanthine into hydrogen peroxide and uric acid, and contacting the reaction liquid with a test strip impregnated with N-ethyl-N-sulfobutyl-m-toluidine, thus developing a color in the test strip; and
(iv) a step for comparing the color developed in the test strip of step (ii) and the color developed in the test strip of step (iii); as well as a measurement kit used in the method.

## Brief Description of the Drawings

Fig. 1 is a drawing showing an example of the processes of the method of the present invention.

Fig. 2 is a graph showing the relationship between the reflectance ratio obtained by the method of Example 1 of the present invention and the absorbance ratio obtained by a conventional method.

Fig. 3 is a drawing showing a second embodiment of the method of the present invention.

Fig. 4 is a drawing of a composite test strip used in the method of the present invention.

Fig. 5 is a drawing showing a calibration curve obtained in Example 3 of the present invention.

Fig. 6 is a drawing showing a third embodiment of the method of the present invention.

## Best Mode for Carrying Out the Invention

The test sample containing ATP-related compounds used in step (i) of the method of the present invention may be prepared by a commonly known method wherein a protein denaturant is added, for example, to a seafood or a meat and, after grinding, the product is filtered. Another acceptable method is to simply homogenize the seafood or meat in a buffer solution. As a protein denaturant, trichloroacetic acid, ethanol, perchloric acid, or the like can be used. To 50 to 100 mg of the sample, 1.25 to 2.50 mg of the protein denaturant is used. The test sample is neutralized in step (i) to a pH of about 6.5 to 8.0 with a neutralizer. Examples of neutralizers which can be used are pH adjusting reagents such as carbonate buffer solution, potassium hydroxide, potassium phosphate, potassium acetate, and the like.

The enzymes used in step (ii) of the present invention are enzymes which can decompose all the ATP-related compounds contained in the test sample to $H_2O_2$ and uric acid. Among these, there must be included alkali phosphatase, adenosine diaminase, nucleotide phosphorylase, and xanthine oxidase. Also, the enzymes used in step (iii) are those which decompose inosine and hypoxanthine among the ATP-related compounds to $H_2O_2$ and uric acid. Nucleotide phosphorylase and xanthine oxidase must be included, and alkali phosphatase and adenosine diaminase must be excluded.

For 50 to 100 mg of the test sample, the enzymes in the enzyme reaction of step (ii) are used in the amounts of 218 to 438 U of alkali-phosphatase, 31 to 63 U of adenosine diaminase, 0.25 to 0.5 U of xanthine oxidase, and 1.25 to 2.5 U of nucleotide phosphorylase, while the enzymes in the enzyme reaction of step (iii) are used in the amounts of 1.25 to 2.5 U of nucleotide phosphorylase and 0.25 to 0.5 U of xanthine oxidase.

To prepare the test strips used in both these steps, a carrier is impregnated with the color developer N-ethyl-N-sulfobutyl-m-toluidine dissolved in water, after which the carrier is dried. Filter paper can be used as a carrier as well as non-woven fabric, gelatine film, collagen film, and the like. An example of preferable methods for preparing the test strip is as follows. 230 to 460 mg of potassium phosphate, 43 to 86 mg of disodium phosphate, and 13 to 24 mg of sodium N-ethyl-N-sulfobutyl-m-toluidine are dissolved in 2.5 to 3.0 ml of distilled water. The carrier is impregnated with this solution by a conventional method, after which it is dried. The dried carrier is cut into pieces of a suitable size and attached by adhesive to a stick-shaped plastic as required. The color development of the test strip in steps (ii) and (iii) is carried out by immersing the test strip in the neutralized sample which has been treated with the respective enzyme. The color developed in step (ii) shows the total amount of ATP-related compounds, while the color developed in step (iii) shows the amount of decomposed inosine and hypoxanthine. As a developer for N-ethyl-N-sulfobutyl-m-toluidine, 4-aminoantipyrine is preferable. The 4-aminoantipyrine can be added during the enzyme reaction in steps (ii) and (iii), but for stability it is preferable that the addition be made during the neutralization in step (i).

In step (iv), the comparison of the amount of color in the test strips from step (ii) and step (iii) is carried out by checking the reflectance of the color phase of the colored strip. Specifically, this can be performed by measuring the reflectance of the test strips obtained from step (ii) and step (iii) after removing the adhering moisture by blotting paper or the like, but it can also be performed by measuring the reflectance without removing the adhering moisture. The freshness of the seafood or meat is judged by the result of the comparison. Specifically, the ratio of the amount of the decomposed inosine and hypoxanthine product (B) to the total amount of ATP-related compounds (A+B) is obtained and expressed as the K ratio. If the K ratio approaches zero, the degree of freshness can be judged as very high. However, if the K ratio approaches 100%, the degree of freshness can be judged as very low.

In the case where the reflectance is measured with the adhering moisture removed from the test strip, the K value may be determined from the reflectance ratio $[(B)/(A+B)]$. If the reflectance is measured without removing the adhering moisture from the test strip, the absolute reflectance $(R\infty)$ is obtained from the Kubelka-Munk equation [*Monthly Pharmaceutical Times,* vol. 26, No. 2, 301 (1984)]. The K/S value is obtained from Equation (1) and the K value then obtained from Equation (2).

$$K/S = (1 - R\infty)^2/2R\infty \quad (1)$$

$$K = \frac{(K/S)_B - (K/S)\,Blank_{(B)}}{(K/S)_{(A+B)} - (K/S)\,Blank_{(A+B)}} \quad (2)$$

The above-mentioned neutralizers and enzyme preparations may be freeze dried in test tubes to improve the stability of the reagents and provide easy usage. When they are freeze dried, a neutralizer, for example, dissolved in a pH adjusting reagent, together with Ficoll and 4-aminoantipyrine as a developer, is charged into a test tube and freeze dried according to a conventional method. Also, for the enzyme preparations, the enzyme as well as optional components, such as Ficoll and the like, may be dissolved in distilled water, charged into test tubes, and freeze dried in the same manner as for the neutralizer. For the enzyme preparations, all the required enzymes may be mixed together in one preparation. These enzymes are optionally mixed with the neutralizers in one preparation. Alternatively, preparations comprising any number of enzymes packaged separately may be used.

The following table gives the embodiments of the present invention along with the reagents and measuring methods used.

| Reagents | First Embodiment | Second Embodiment | Third Embodiment |
|---|---|---|---|
| A | 4-AA + Neutralizer | 4-AA + Neutralizer | 4-AA + Neutralizer |
| B | Alkali phosphatase<br>Adenosine diaminase | Alkali phosphatase<br>Adenosine diaminase | Alkali phosphatase<br>Adenosine diaminase<br>4-AA + Neutralizer |
| C | Nucleotide phosphorylase<br>Xanthine oxidase<br>Peroxidase | Composite test strip<br>[Nucleotide phosphorylase<br>Xanthine oxidase<br>Peroxidase]  (impregnated) | Composite test strip<br>[Nucleotide phosphorylase<br>Xanthine oxidase<br>Peroxidase]  (impregnated) |
| D. | Test strip<br>N-ethyl-N-sulfobutyl-<br>m-toluidine (impregnated) | +<br>N-ethyl-N-sulfobutyl-<br>m-toluidine (impregnated) | +<br>N-ethyl-N-sulfobutyl-<br>m-toluidine (impregnated) |
| Procedure | Add A → Add B → Add C → Immerse D<br>(Add C → Immerse D) | Add A → Add B → Immerse D<br>(Immerse D) | Add A → Add B → Immerse D<br>(Immerse D) |

In the first embodiment of the present invention, A is the reagent used for neutralization in step (i). Sodium carbonate buffer solution or the like is used as the neutralizer. Among enzyme preparations, B is used in step (ii) and C is used in step (iii).

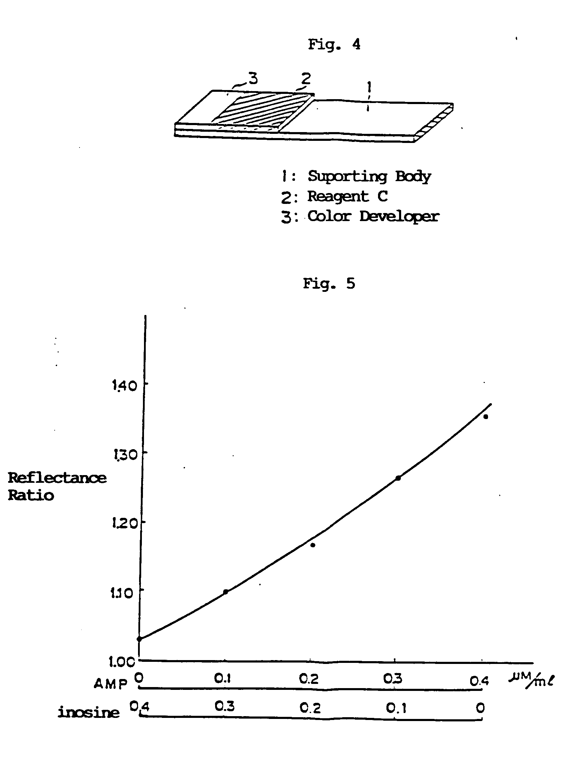

As shown in Fig. 4, in the second and third embodiments of the present invention, composite test strips in which the C reagent and the color developer are impregnated in carriers made of the identical material are used. The test strips used in these embodiments are made up of zones in sheet form which are in mutual contact for adsorption via the tips of the test strips, wherein the enzyme reaction components (C reagent) and the color developing component are arranged so that they are separately present. These test strips are then affixed to a rigid carrier. On immersion in the test sample, the liquid, controlled by the capillarity, passes through the test strip containing the color developer and finally the test strip with the reagent C develops a color.

A preferred example of the method for preparing the test strips is as follows. 5.0 to 10.5 mg of potassium phosphate, 8.5 to 17.5 mg of sodium phosphate, 125 to 250 U of peroxidase, 0.3 to 0.6 U of xanthine oxidase, 1.5 to 3 U of nucleotide phosphorylase, 100 to 200 mg of Ficoll, and 40 to 85 mg of sucrose are dissolved in 1.5 to 2.0 ml of distilled water, a carrier is impregnated with this solution in the usual manner, and then dried. The dried carrier is cut into suitably sized pieces as reagent C test strips. Then 230 to 460 mg of potassium phosphate, 43 to 86 mg of disodium phosphate, and 12 to 24 mg of sodium N-ethyl-N-sulfobutyl-m-toluidine are dissolved in 0.8 to 1.0 ml of distilled water, a carrier is impregnated with this solution, and dried in the usual manner. The dried carrier is cut into suitably sized pieces for use as color developer test strips, and these, along with the previously-obtained reagent C test strips, are attached by adhesive to a plastic stick or the like in juxtaposition.

The use of the composite test strip ensures the addition of the reagent C to the test sample and the immersion of the color developing strip at the same time, thus providing an easier method of measuring the freshness of seafood or meat.

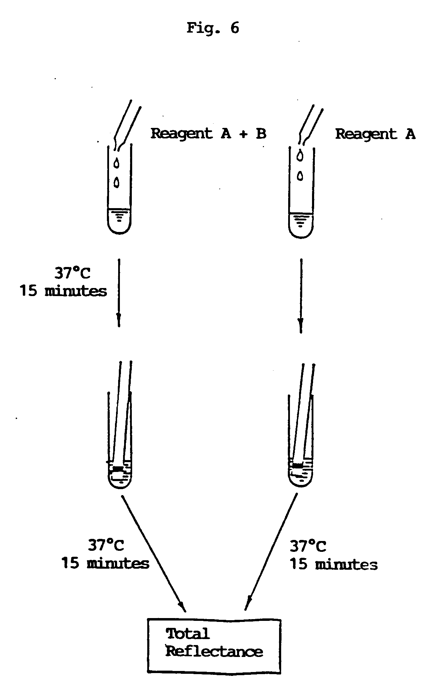

Among the methods of using the composite test strips, the third embodiment integrates the step of neutralizing the test samples containing ATP-related compounds and the step of reacting with enzymes to decompose the sample into inosine and hypoxanthine for simplicity of the process. This will now be explained with reference to a specific example. 20 to 40.5 mg of 4-aminoantipyrine and 200 to 400 mg of Ficoll are dissolved in 16 to 20 ml of a 0.2 M carbonate buffer solution. This solution is charged into test tubes in 100 µl lots and freeze dried for measurement of the inosine and hypoxanthine decomposition components. Separately, 20 to 40.5 mg of 4-aminoantipyrine, 200 to 400 mg of Ficoll, 525 to 1,050 U of a freeze dried alkali phosphatase product, and 75 to 150 U of a freeze dried adenosine diaminase product are dissolved in 16 to 20 ml of a 0.2 M carbonate buffer solution. This solution is charged into test tubes in 100 µl lots and freeze dried for measurement of the total amount of ATP-related compounds. As shown in Fig. 6, the test sample is directly added to a test tube for measurement of the total amount of ATP-related compounds and a test tube for measurement of the inosine and hypoxanthine decomposition components, and the respective test strips can be immersed simultaneously in the appropriate solutions. This results in an even simpler method of measuring the freshness of seafood or meat.

The manner by which the method of the present invention is carried out will be specifically explained with reference to an example of a freeze dried reagent of the first embodiment.

To a small amount of a seafood or a meat sample cut into small strips a solution of a protein denaturant is added. The mixture is ground in a mortar or the like and filtered. The filtrate is then added to reagent A in a test tube and the freeze dried reagent is dissolved. A prescribed amount of this liquid is added to reagent B in a test tube, and incubated for several minutes to several tens of minutes at room temperature to 37°C.

Two test tubes of reagent C are prepared. An amount of liquid equivalent to that previously taken from the test tube of reagent A is taken from the remainder in the test tube and added to one of the test tubes of reagent C. The previously incubated solution is added to the other test tube of reagent C. Then, test strips prepared by the method outlined above are immersed in these test tubes and incubated for several minutes to several tens of minutes at the room temperature to 37°C range, after which the reflectance of the color of the respective test strips is determined, and the K value calculated.

The freshness of the seafood or meat can be easily judged in this manner.

Examples

The following examples are given for detailed explanation purposes and are in no way restrictive of the present invention.

Example 1

(1) Reagent preparation

[1] Reagent A (neutralizer)

81 mg of 4-aminoantipyrine and 800 mg of Ficoll 400 were dissolved in 40 ml of 0.2 M carbonate buffer solution. This solution was charged into test tubes in 200 µl lots and freeze dried. After freeze drying, the test tube was flushed with $N_2$ gas and tightly capped.

[2] Reagent B

525 U of an alkali phosphatase freeze dried product, 75 U of an adenosine diaminase freeze dried product, and 200 mg of Ficoll 400 were dissolved in 10 ml of distilled water. This solution was charged into test tubes in 100 $\mu$l lots and freeze dried. After freeze drying, the test tube was flushed with $N_2$ gas and tightly capped.

[3] Reagent C

42 mg of potassium phosphate, 70 mg of disodium phosphate, 800 U of peroxidase, 2.4 U of xanthine oxidase, 12 U of nucleotide phosphorylase, 800 mg of Ficoll, and 340 mg of sucrose were dissolved in 40 ml of distilled water. This solution was charged into test tubes in 100 $\mu$l lots and freeze dried. After freeze drying, the test tube was flushed with $N_2$ gas and tightly capped.

[4] Test strips

230 mg of potassium phosphate, 43 mg of disodium phosphate, and 12 mg of sodium N-ethyl-N-sulfobutyl-m-toluidine were dissolved in 3 ml of distilled water. A sheet of Toyo No. 526 filter paper measuring 6 cm x 6 cm (36 cm²) was impregnated with this solution and dried under vacuum. The dried filter paper was cut into 6 mm x 6 mm squares and attached to one end of a plastic carrier using double sided adhesive tape (Tesaband: trade mark, manufactured by Bayersdorf).

(2) Operating procedures

A test sample was prepared by blending AMP and inosine to give a total concentration of 0.4 $\mu$M/ml. 120 $\mu$l of this solution was added to the reagent A and 60 $\mu$l of the resulting mixture were added to the reagent B and incubated for 15 minutes at 37°C. Two test tubes of the reagent C .PL11.50"were prepared. 60 $\mu$l of the balance of the reagent A were added to one test tube of the reagent C, and 60 ml of the incubated reagent B prepared above were added to the other test tube. The respective test strips were then immersed in the test tubes and incubated for 15 minutes at 37°C. The test strips were removed from the test tubes and the adhering moisture was gently removed with blotting paper, after which the reflectances were measured at 560 nm using a reflectometer. The (A+B) and (B) reflectances were determined and the (B)/(A+B) reflectance ratio was calculated.

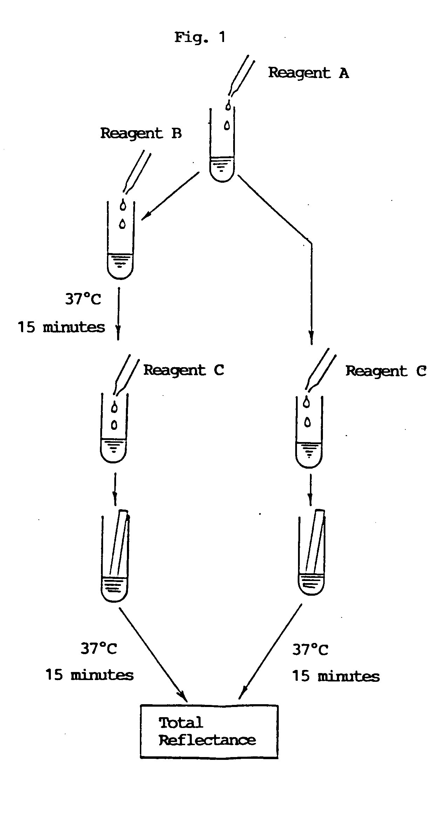

The outline of these procedures is shown in Fig. 1.

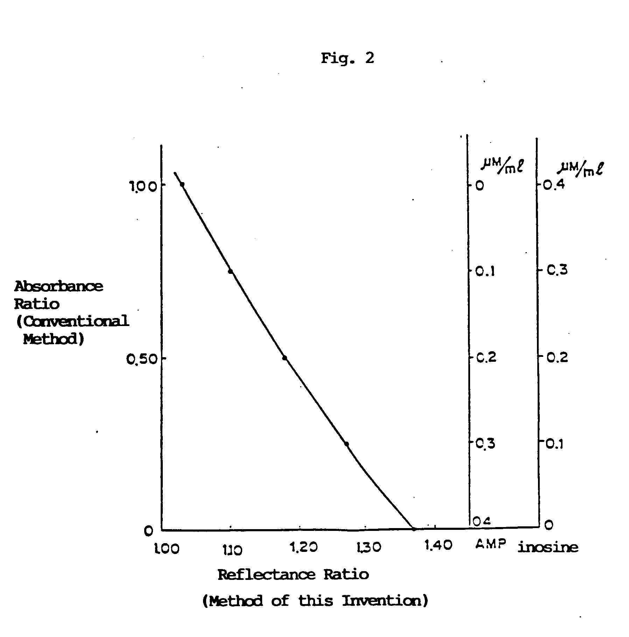

The relationship between the reflectance ratios obtained in this manner and the absorption ratio from a conventional method were examined. The results are shown in Fig. 2. To obtain the absorption ratios, instead of using a test strip, 200 $\mu$l of a color developing liquid of the following formulation was poured into a test tube containing freeze dried reagent C at the time when the test strip would have been immersed, and after incubating for 15 minutes at 37°C the absorbance was measured to determine (A+B) and (B) respectively. The absorption ratio (B)/(A+B) was calculated.

| | |
|---|---|
| **Potassium phosphate** | **230 mg** |
| **Sodium phosphate** | **43 mg** |
| **Sodium N-ethyl-N-sulfobutyl-m-toluidine** | **12 mg** |
| **Purified water** | **20 ml** |

As can be clearly seen from Fig. 2, the present method shows good correlation with the conventional method and can obviously be used to measure the K value.

Example 2

Phenol, dimethylaniline, dimethyl-m-toluidine, and N-ethyl-N-sulfobutyl-m-toluidine were used as the color developer. The test strip method of the present invention and a commonly known color development liquid method were carried out to compare the difference in sensitivity due to difference in the color developer. As a sample, equal amounts (50 μl) of preparations containing 0.2 μM/ml inosine and 0.2 μM/ml AMP were used. The commonly known color development liquid had the formulation shown below. As the operating method, instead of using a test strip, 200 μl of the color development liquid was charged into a test tube containing freeze dried reagent C, and, after incubation, the absorbance was measured at 500 nm.

The test strip method was carried out in the same manner as in Example 1 with the exception that the color change was judged visually [white (-) ↔ purple (+++)]. The results are given in Table 1.

| Potassium phosphate | 230 mg |
|---|---|
| Sodium phosphate | 43 mg |
| Phenol | 283.2 mg, or |
| Dimethylaniline | 363.5 mg, or |
| Diethyl-m-toluidine | 405 mg |
| Purified water | 20 ml |

## TABLE 1

| Color Coupler | Color Development Liquid (Absorbance) | | Test Strip Method (Color) |
|---|---|---|---|
| Phenol | | | |
| | $\dfrac{(B)}{(A+B)}$ = | $\dfrac{0.004}{0.004}$ | (B)/(A+B)= White/White = (-)/(-) |
| Dimethylaniline | | | |
| | $\dfrac{(B)}{(A+B)}$ = | $\dfrac{0.013}{0.017}$ | (B)/(A+B)= White/White = (±)/(+) |
| Diethyl-m-toluidine | | | |
| | $\dfrac{(B)}{(A+B)}$ = | $\dfrac{0.017}{0.027}$ | (B)/(A+B) = Very light purple/ Light purple = (+)/(++) |
| Sodium N-ethyl-N-sulfobutyl-m-toluidine | | | |
| | $\dfrac{(B)}{(A+B)}$ = | $\dfrac{0.018}{0.042}$ | (B)/(A+B) = Light purple/Purple = (+)/(+++) |

As shown in Table 1, for both the color development liquid method and the test strip method, the sensitivity of phenol, dimethylaniline, and diethyl-m-toluidine was judged to be clearly inferior to that of sodium N-ethyl-N-sulfobutyl-m-toluidine.

Example 3

(1) Reagent preparation

[1] Reagent A (neutralizer)

Same as Example 1.

[2] Reagent B

Same as Example 1.

[3] Reagent C

10.5 mg of potassium phosphate, 17.5 mg of disodium phosphate, 200 U of peroxidase, 0.6 U of xanthine oxidase, 3 U of nucleotide phosphorylase, 200 mg of Ficoll 400, and 85 mg of sucrose were dissolved in 1.8 ml of distilled water. A sheet of Toyo No. 526 filter paper measuring 6 cm x 6 cm was impregnated with this solution and dried under vacuum. The dried filter paper was cut into 6 mm x 6 mm squares

[4] Color developer test strips

230 mg of potassium phosphate, 43 mg of disodium phosphate, and 12 mg of sodium N-ethyl-N-sulfobutyl-m-toluidine were dissolved in 1 ml of distilled water. A sheet of Toyo No. 526 filter paper measuring 6 cm x 3 cm was impregnated with this solution and dried under vacuum. The dried filter paper was cut into 6 mm x 3 mm rectangles.

The test strips [3] and [4] were alternately attached to a plastic carrier using double sided adhesive tape (Tesaband; trade mark produced by Bayersdorf) to form a composite test strip 6 mm wide x 9 mm long. Fig. 4 is a perspective drawing of this composite strip.

(2) Operating procedures

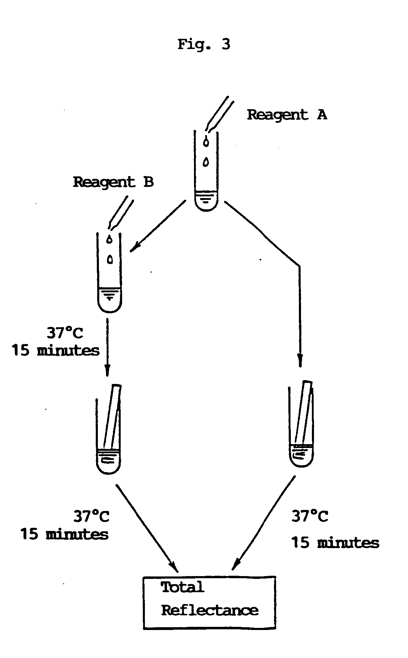

A test sample was prepared by blending AMP and inosine to give a total concentration of 0.4 $\mu$M/ml. 120 $\mu$l of this solution was added to the reagent A and 60 $\mu$l of the resulting mixture was added to the reagent B and incubated for 15 minutes at 37°C. Two composite test strips were prepared, combining test strips impregnated with the reagent C and the color developer respectively. The respective test strips were then immersed in the balance of the reagent A and the incubated reagent B prepared above, and incubated for 15 minutes at 37°C. The test strips were removed from the test tubes and the adhering moisture was gently removed with blotting paper, after which the reflectances were measured at 560 nm using a reflectometer. The (A+B) and (B) reflectances were obtained and the (B)/(A+B) reflectance ratio was calculated. The outline of these operating procedures is shown in Fig. 3. The reflectance ratios obtained in this manner are shown in Fig. 5.

Example 4

(1) Reagent preparation

[1] Reagent A

40.5 mg of 4-aminoantipyrine and 400 mg of Ficoll 400 were dissolved in 20 ml of 0.2 M carbonate buffer solution. This solution was charged into test tubes in 100 $\mu$l lots and freeze dried. After freeze drying, the test tube was flushed with $N_2$ gas and tightly capped.

[2] Reagent A+B

40.5 mg of 4-aminoantipyrine, 400 mg of Ficoll 400, 1,050 U of an alkali phosphatase freeze dried product, and 150 U of an adenosine diaminase freeze dried product were dissolved in 19.4 ml of 0.2 M carbonate buffer solution. This solution was charged into test tubes in 100 $\mu$l lots and freeze dried. After freeze drying the test tube was flushed with $N_2$ gas and tightly capped.

[3] Reagent C

Same as Example 3.

[4] Test strips

Same as Example 3.

(2) Operating procedures

A test sample was prepared by blending AMP and inosine to give a total concentration of 0.4 $\mu$M/ml. 60 $\mu$l of this solution were added to the reagent A and to the reagent A+B respectively. The reagent A+B with the added sample was incubated for 15 minutes at 37°C. Two composite test strips were prepared, combining test strips impregnated with the reagent C and the color developer respectively. The respective composite test strips were then immersed in the reagent A and in the incubated reagent A+B prepared above, which was incubated for 15 minutes at 37°C. The composite test strips were removed from the test tubes and the adhering moisture was gently removed with blotting paper, after which the reflectances were measured at 560 nm using a reflectometer. The (A+B) and (B) reflectances were obtained and the (B)/(A+B) reflectance ratio was calculated. The outline of these operating procedures is shown in Fig. 6. The results of the reflectance ratios obtained in this manner were the same as the results obtained in Example 3.

Example 5

(1) Reagent preparation

Reagent A, reagent A+B, the reagent C test strips, and the color developer test strips were all prepared in the same manner as in Example 4.

(2) Operating procedures

Operating procedures were the same as in Example 4, except that the composite test strips removed from the test tubes were subjected to measurement of the reflectances at 560 nm using a spectral color difference meter [Sigma ($\Sigma$)-80: trade mark, produced by Nihon Denshoku Kogyo K.K.], without removing the adhering moisture. $(K/S)_{(A+B)}$, $(K/S)_B$, $(K/S)$ Blank$_{(A+B)}$, and $(K/S)$ Blank$_{(B)}$ were obtained from Equation 1, and the K value was calculated from Equation 2. The results are shown in Table 2.

## TABLE 2

| A+B (AMP) | B (Inosine) | (A+B) R∞ | | B R∞ | | K va |
|---|---|---|---|---|---|---|
| | | Measured | Blank value | Measured | Blank value | |
| μM/ml | μM/ml | % | % | % | % | % |
| 0.40 | 0 | 38.12 | 59.19 | 55.60 | 58.68 | 2.0 |
| 0.36 | 0.04 | 33.62 | 59.19 | 55.54 | 58.68 | 14.0 |
| 0.32 | 0.08 | 38.06 | 59.19 | 49.96 | 58.68 | 23.0 |
| 0.28 | 0.12 | 33.37 | 59.19 | 45.13 | 58.68 | 34.6 |
| 0.24 | 0.16 | 32.53 | 59.19 | 43.21 | 58.68 | 40.2 |
| 0.20 | 0.20 | 35.07 | 59.19 | 42.70 | 58.68 | 52.4 |
| 0.10 | 0.30 | 31.55 | 59.19 | 36.45 | 58.68 | 72.3 |
| 0 | 0.40 | 30.90 | 59.19 | 33.58 | 58.68 | 87.9 |

Example 6

(1) Reagent preparation

[1] Reagent A

40.5 mg of 4-aminoantipyrine and 400 mg of Ficoll 400 were dissolved in 20 ml of 0.1 M Tris buffer solution. This solution was charged into test tubes in 100 μl lots and freeze dried. After freeze drying the test tube was flushed with $N_2$ gas and tightly capped.

[2] Reagent A+B

40.5 mg of 4-aminoantipyrine, 400 mg of Ficoll 400, 1,050 U of an alkali phosphatase freeze dried product, and 150 U of an adenosine diaminase freeze dried product were dissolved in 19.4 ml of 0.1 M Tris buffer solution. This solution was charged into test tubes in 100 μl lots and freeze dried.
After freeze drying the test tube was flushed with $N_2$ gas and tightly capped.

[3] Reagent C

Same as Example 3.

[4] Test strips

Same as Example 3.

(2) Operating procedures

100 mg of raw tuna fish for *sashimi* were placed in 5 ml of 0.1 M Tris buffer solution and the mixture was homogenized. 60 ml of this homogenized mixture were added to the reagent A and to the reagent A+B respectively. The reagent A+B with the added sample was incubated for 15 minutes at 37°C. Two composite test strips were prepared, combining test strips impregnated with the reagent C and the color developer respectively. The respective composite test strips were then immersed in the reagent A and the incubated reagent A+B prepared above, and incubated for 15 minutes at 37°C. The composite test strips were removed from the test tubes and the reflectances were measured at 560 nm using a spectral color difference meter, without removing the adhering moisture from the strips. The K values were obtained in the same manner as in Example 5.

For comparison purposes, the same procedure as above was performed on tne test solution prepared by a commonly known method, in which the protein was removed by trichloroacetic acid instead of homogenizing the tuna *sashimi* in 0.1 M Tris buffer solution.

The results are shown in Table 3. The results obtained by both samples were in good agreement.

## TABLE 3

|  | Treated with trichloroacetic acid | Homogenized with Tris buffer |
|---|---|---|
| K value (%) 1 | 5.46 | 4.29 |
| K value (%) 2 | 4.30 | 5.83 |
| K value (%) 3 | 4.67 | 6.00 |

Industrial Utilization

The method of the present invention utilizes N-ethyl-N-sulfobutyl-m-toluidine as a color developer which can give high sensitivity and which can be dried, and utilizes a test strip impregnated with N-ethyl-N-sulfobutyl-m-toluidine or a composite test strip using both the reagent C and this color developer. Therefore, the freshness of seafoods and meats can be measured easily and quickly without the necessity for large-scale equipment.

**Claims**

1. A method for measuring freshness of meat or seefoods samples which comprises:
    (i) a step of obtaining a test sample containing ATP-related compounds by adding a protein denaturant to seafoods or meats, and grinding the mixture, followed by extraction;
    (ii) a step for neutralizing the test sample containing ATP-related compounds;
    (iii) a step for reacting a prescribed amount of the test sample neutralized in step (ii) which contains 4-aminoantipyrine and an enzyme which can decompose ATP-related compounds into hydrogen peroxide and uric acid, and contacting the reaction liquid with a test strip impregnated with N-ethyl-N-sulfobutyl-m-toluidine, thus developing a color in the test strip;
    (iv) a step for reacting a prescribed amount of the test sample neutralized in step (ii) which contains 4-aminoantipyrine and an enzyme which can decompose inosine and hypoxanthine into hydrogen peroxide and uric acid, and contacting the reaction liquid with a test strip impregnated with N-ethyl-N-sulfobutyl-m-toluidine, thus developing a color in the test strip; and
    (v) a step for comparing the color developed in the test strip of step (iii) and the color developed in the test strip of step (iv).

2. The method according to Claim 1, wherein the neutralization in step (ii) is accomplished by a neutralizer also containing 4-aminoantipyrine.

3. The method according to Claim 1, wherein the enzyme in process step(iii) is a mixture of alkali phosphatase, adenosine diaminase, nucleotide phosphorylase, and xanthine oxidase.

4. The method according to Claim 1, wherein the enzyme in step (iv) is a mixture of nucleotide phosphorylase and xanthine oxidase.

12

5. A kit for measuring freshness of meat or seafoods samples comprising:
(a) a neutralizer containing 4-aminoantipyrine;
(b) an enzyme reagent containing alkali phosphatase and adenosine diaminase;
(c) an enzyme reagent containing nucleotide phosphorylase, xanthine oxidase, and peroxidase; and
(d) a test strip impregnated with N-ethyl-N-sulfobutyl-m-toluidine.

6. A kit according to claim 5, wherein the reactants of (c) and (d) of claim 5 are combined into a composite test strip comprising a test strip impregnated with nucleotide phosphorylase, xanthine oxidase and peroxidase and a test strip impregnated with N-ethyl-N-sulfobutyl-m-toluidine.

7. A kit according to claim 5 comprising:
(a) a neutralizer containing 4-aminoantipyrine;
(b) a neutralizer and enzyme reagent containing 4-aminoantipyrine, alkali-phosphatase, and adenosine diaminase; and
(c) a composite test strip comprising a test strip impregnated with nucleotide phosphorylase, xanthine oxidase, and peroxidase and a test strip impregnated with N-ethyl-N-sulfobutyl-m-toluidine.

**Patentansprüche**

1. Verfahren zum Messen der Frische von Fleischproben oder Proben eßbarer Meerestiere, umfassend:
(i) eine Stufe des Herstellens einer Testprobe, die zu ATP in Beziehung stehende Verbindungen enthält, durch Zugabe eines Protein-Denaturierungsmittels zu eßbaren Meerestieren oder Fleisch und Mahlen der Mischung, gefolgt von einer Extraktion;
(ii) eine Stufe des Neutralisierens der Testprobe, die zu ATP in Beziehung stehende Verbindungen enthält;
(iii) eine Stufe des Umsetzens einer vorgeschriebenen Menge der in Stufe (ii) neutralisierten Testprobe, die 4-Aminoantipyrin und ein Enzym enthält, das zu ATP in Beziehung stehende Verbindungen zu Wasserstoffperoxid und Harnsäure zersetzen kann und Kontaktieren der Reaktionsflüssigkeit mit einem Teststreifen, der mit N-Ethyl-N-sulfobutyl-m-toluidin imprägniert ist, wodurch eine Farbe in dem Teststreifen entwickelt wird;
(iv) eine Stufe des Umsetzens einer vorgeschriebenen Menge der in Stufe (ii) neutralisierten Testprobe, die 4-Aminoantipyrin und ein Enzym enthält, das Inosin und Hypoxanthin in Wasserstoffperoxid und Harnsäure zersetzen kann, und in Berührung bringen der Reaktionsflüssigkeit mit einem Teststreifen, der mit N-Ethyl-N-sulfobutyl-m-toluidin imprägniert ist, wodurch eine Farbe im Teststreifen entwickelt wird, und
(v) eine Stufe des Vergleichens der im Teststreifen der Stufe (iii) entwickelten Farbe und der Teststreifen der Stufe (iv) entwickelten Farbe.

2. Verfahren nach Anspruch 1, worin die Neutralisatron in Stufe (ii) mittels eines Neutralisationsmittels ausgeführt wird, das auch 4-Aminoantipyrin enthält.

3. Verfahren nach Anspruch 1, worin das Enzym in Stufe (iii) eine Mischung von Alkaliphosphatase, Adenosin-Diaminase, Nucleotid-Phosphorylase und Xanthin-Oxidase ist.

4. Verfahren nach Anspruch 1, worin das Enzym in Stufe (iv) eine Mischung von Nucleotid-Phosphorylase und Xanthin-Oxidase ist.

5. Ausrüstung zum Messen der Frische von Fleischproben oder Proben eßbarer Meerestiere, umfassend:
(a) ein 4-Aminoantipyrin enthaltendes Neutralisationsmittel;
(b) ein Alkaliphosphatase und Adenosin-Diaminase enthaltendes Enzym-Reagenz;
(c) ein Nucleotid-Phophorylase, Xanthin-Oxidase und Peroxidase enthaltendes Enzym-Reagenz und
(d) einen mit N-Ethyl-N-sulfobutyl-m-toluidin imprägnierten Teststreifen.

6. Ausrüstung nach Anspruch 5, worin die Reagenzien von (c) und (d) nach Anspruch 5 zu einem Verbund-Teststreifen kombiniert sind, der einen mit Nucleotid-Phosphorylase, Xanthin-Oxidase und Peroxidase imprägnierten Teststreifen und einen mit N-Ethyl-N-sulfobutyl-m-toluidin imprägnierten Teststreifen umfaßt.

7. Ausrüstung nach Anspruch 5, umfassend:
   (a) ein 4-Aminoantipyrin enthaltendes Neutralisationsmittel;
   (b) ein Neutralisationsmittel und 4-Aminoantipyrin, Alkaliphosphatase und Adenosin-Diaminase enthaltendes Enzym-Reagenz und
   (c) einen Verbund-Teststreifen, umfassend einem mit Nucleotid-Phosphorylase, Xanthin-Oxidase und Peroxidase imprägnierten Teststreifen und einen mit N-Ethyl-N-sulfobutyl-m-toluidin imprägnierten Teststreifen.

## Revendications

1. Procédé pour mesurer la fraîcheur d'échantillons de produits carnés ou de pêche qui comprend:
   (i) une étape de prélèvement d'un échantillon d'analyse contenant des composés apparentés à l'ATP par adjonction d'un dénaturant de protéine aux produits carnés ou de pêche, et le broyage du mélange suivi d'extraction;
   (ii) une étape de neutralisation de l'échantillon d'analyse contenant des composés apparentés à l'ATP;
   (iii) une étape consistant à faire réagir une quantité prescrite de l'échantillon d'analyse neutralisé à l'étape (ii) qui renferme de l'amino-4-antipyrine avec une enzyme capable de décomposer les composés apparentés à l'ATP en peroxyde d'hydrogène et en acide urique, et à mettre en contact le milieu réactionnel liquide avec une bandelette d'analyse imprégnée de N-éthyl-N-sulfobutyl-m-toluidine, de manière à développer une couleur sur la bandelette d'analyse;
   (iv) une étape consistant à faire réagir une quantité prescrite de l'échantillon d'analyse neutralisé à l'étape (ii) qui renferme de l'amino-4-antipyrine avec une enzyme capable de décomposer l'inosine et l'hypoxanthine en peroxyde d'hydrogène et en acide urique, et à mettre en contact le milieu réactionnel liquide avec une bandelette d'analyse imprégnée de N-éthyl-N-sulfobutyl-m-toluidine de manière à développer une couleur sur la bandelette d'analyse; et
   (v) une étape consistant à comparer la couleur qui s'est développée sur la bandelette d'analyse au cours de l'étape (iii) à la couleur qui s'est développée sur la bandelette d'analyse au cours de l'étape (iv).

2. Procédé selon la revendication 1, dans lequel l'étape de neutralisation (ii) est conduite au moyen d'un neutralisant contenant également de l'amino-4-antipyrine.

3. Procédé selon la revendication 1, dans lequel l'enzyme employée au cours de l'étape (iii) est un mélange de phosphatase alcaline, d'adénosine désaminase, de nucléotide phosphorylase, et de xanthine oxydase.

4. Procédé selon la revendication 1, dans lequel l'enzyme employée au cours de l'étape (iv) est un mélange de nucléotide phosphorylase et de xanthine oxydase.

5. Trousse pour mesurer la fraîcheur d'échantillons de produits carnés ou de pêche comprenant:
   (a) un neutralisant contenant de l'amino-4-antipyrine;
   (b) un réactif enzymatique contenant de la phosphatase alcaline et de l'adénosine désaminase;
   (c) un réactif enzymatique contenant de la nucléotide phosphorylase, de la xanthine oxydase et de la peroxydase; et
   (d) une bandelette d'analyse imprégnée de N-éthyl-N-sulfobutyl-m-toluidine.

6. Trousse selon la revendication 5, dans laquelle les réactifs (c) (d) selon la revendication 5 sont réunis dans une bandelette d'analyse composite comprenant une bandelette d'analyse imprégnée de nucléotide phosphorylase, de xanthine oxydase et de peroxydase et une bandelette d'analyse imprégnée de N-éthyl-N-sulfobutyl-m-toluidine.

7. Trousse selon la revendication 5 comprenant:
   (a) un neutralisant contenant de l'amino-4-antipyrine;
   (b) un neutralisant et un réactif enzymatique contenant de l'amino-4-antipyrine, de la phosphatase alcaline et de l'adénosine désaminase; et
   (c) une bandelette d'analyse composite comprenant une bandelette d'analyse imprégnée de nucléotide phosphorylase, de xanthine oxydase et de peroxydase et une bandelette d'analyse imprégnée de N-éthyl-N-sulfobutyl-m-toluidine.

Fig. 1

Fig. 2

Reflectance Ratio
(Method of this Invention)

Fig. 3

Reagent A

Reagent B

37°C
15 minutes

37°C
15 minutes

37°C
15 minutes

Total
Reflectance

Fig. 4

1: Suporting Body
2: Reagent C
3: Color Developer

Fig. 5

Fig. 6